# EUROPEAN PATENT APPLICATION

(11) **EP 4 582 328 A1**
(43) Date of publication of application: **09.07.2025**
(21) Application number: 24150207.9
(22) Date of filing: 03.01.2024
(51) Int. Cl.: B61L 15/00, A61B 5/18, G02B 27/01

(54) **DRIVER-ASSIST SYSTEM FOR MONITORING OPERATIONS OF A TRAIN**

(71) Applicant: Siemens Mobility GmbH, 81739 München (DE)
(72) Inventor: JOSHI, Girish, 411004 Pune, Maharashtra (IN); MADIA, Naman, 400080 Mumbai (IN)
(74) Representative: Isarpatent

(57) **Abstract**

A driver-assist system (100) for monitoring operations of a train is disclosed herein. The driver-assist system (100) comprises a heads-up display (105) suitable for wearing by a driver of a train, and an equipment onboard the train comprising one or more communication interfaces (185) for enabling connection with a heads-up display (105) and one or more subsystems (190) onboard the train. The onboard equipment (110) comprises a memory (180), and a processing unit (175) configured to execute one or more instructions stored in the memory (180), to process operational data obtained from at least one of the subsystems to determine safety-critical data, to generate one or more commands based on the safety-critical data determined, and to transmit the one or more commands for execution to the heads-up display (105). The execution of the one or more commands cause the heads-up display (105) to display safety-critical data associated with the one or more subsystems (190) in real-time.

## Description

The present invention relates to monitoring operations of a train. More particularly, the present invention relates to a driver-assist system for monitoring operations of a train.

Conventionally, a driver of a train must continuously monitor a Driver-Machine Interface (DMI) provided inside the locomotive of the train, for critical information, alerts, warnings, that are pertinent to the operation of the train. Additionally, the driver must also keep an eye on the railway track via the windshield, to watch out for potential hazards such as animals, humans or vehicles that may be present on or near the track. This helps the driver to operate the train horn or brake at the right time to warn the animal, human or vehicle on the track, or to stop an oncoming train. The driver must also watch out for damaged tracks, landslides, other trains or train wrecks ahead of the train to avoid potential disasters. Consequently, the driver must constantly shift his gaze to focus on the DMI and the track simultaneously. However, such constant shifting of the gaze between the DMI and the windscreen may cause the driver to miss critical information shown on the DMI. This may lead to inaction or delayed reactions from the driver, thereby leading to accidents. Furthermore, in case of trains (locomotives) which consist of multiple Onboard equipment (OBUs), the driver may be required to change their seating position after every OBU change.

In light of the above, there exists a need for a system that enables the driver to monitor operations of a train, without the need for constantly watching the DMI.

Therefore, it is an object of the invention to provide an onboard equipment and a driver-assist system for monitoring operations of a train.

In an aspect of the present invention, an onboard equipment configured for mounting inside a cabin of a train is disclosed. The onboard equipment includes one or more communication interfaces for enabling connection with a heads-up display and one or more subsystems onboard the train. The onboard equipment further includes a memory, and a processing unit configured to execute one or more instructions stored in the memory, to: process operational data obtained from at least one of the subsystems to determine safety-critical data; generate one or more commands based on the safety-critical data determined; and transmit, to the heads-up display, the one or more commands for execution, wherein the execution of the one or more commands cause the heads-up display to display the safety-critical data in real-time. In an embodiment, the onboard equipment is a Driver-Machine Interface. In another embodiment, the onboard equipment is an onboard vital computer.

In another aspect of the present invention, a driver-assist system is disclosed. The driver-assist system includes a heads-up display, suitable for wearing by a driver of a train, and an onboard equipment. The onboard equipment includes one or more communication interfaces for enabling connection with a heads-up display and one or more subsystems onboard the train. The onboard equipment further includes a memory, and a processing unit configured to execute one or more instructions stored in the memory, to: process operational data obtained from at least one of the subsystems to determine safety-critical data; generate one or more commands based on the safety-critical data determined; and transmit, to the heads-up display, the one or more commands for execution, wherein the execution of the one or more commands causes the heads-up display to display the safety-critical data in real-time. In an embodiment, the onboard equipment is a Driver-Machine Interface. In another embodiment, the onboard equipment is an onboard vital computer.

In an embodiment, the heads-up display includes a data interface for receiving the one or more commands transmitted by the onboard equipment, a translucent display, a controller unit configured to generate visuals indicative of the safety-critical data based on the one or more commands received, and an optics unit configured to display the visuals generated by the controller unit, on the translucent display. In a further embodiment, the heads-up display further includes one or more physiological sensors, and a feedback unit configured to: identify one or more hazards based on one or more physiological signs associated with the driver, detected by the one or more physiological sensors; configure a feedback signal based on the identified one or more hazards; and concurrently output the feedback signal with the visuals on the translucent display. In an embodiment, the feedback signal comprises at least one of a tactile signal and an audio signal.

In yet another aspect, a method for enabling a driver-assist system is disclosed. The method includes processing, by an onboard equipment mounted inside a cabin of a train, operational data obtained from at least one subsystem onboard the train to determine safety-critical data. The method further includes generating, by the onboard equipment, one or more commands based on the safety-critical data determined; and transmitting, by the onboard equipment, the one or more commands for execution, to a heads-up display. The execution of the one or more commands causes the heads-up display (105) to display the safety-critical data in real-time. In an embodiment, the method further includes generating, by the heads-up display, visuals indicative of safety-critical data, by the heads-up display based on the one or more commands received from the onboard equipment. The method may further include identifying, by the heads-up display, one or more hazards based on one or more physiological signs associated with the driver. Further, a feedback signal is configured, by the heads-up display, based on the identified one or more hazards. The feedback signal is concurrently outputted with the visuals, by the heads-up display. In an embodiment, the feedback signal comprises at least one of a tactile signal and an audio signal.

The above-mentioned attributes, features, and advantages of the present invention and the manner of achieving them, will become more apparent and understandable (clear) with the following description of embodiments of the invention in conjunction with the corresponding drawings. The illustrated embodiments are intended to illustrate, but not limit the invention.

The present invention is further described hereinafter with reference to illustrated embodiments shown in the accompanying drawings, in which:
- FIG 1A: shows a driver-assist system for enabling a driver to monitor operations of a train, in accordance with an embodiment of the present invention;
- FIG 1B: illustrates functional blocks of a heads-up display, in accordance with an embodiment of the present invention;
- FIG 2: shows a flowchart of a method for enabling the driverassist system for monitoring operations of a train in real-time, in accordance with an embodiment of the present invention; and
- FIG 3: shows an exemplary illustration of visuals displayed on the heads-up display.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth to provide a thorough understanding of one or more embodiments. It may be evident that such embodiments may be practiced without these specific details.

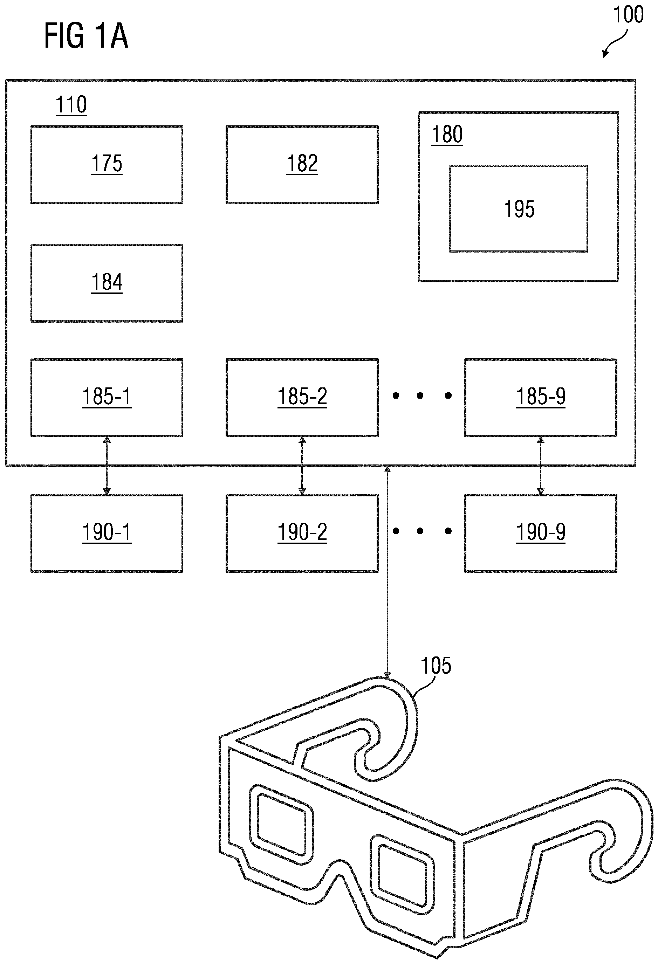

FIG 1A illustrates functional blocks of a driver-assist system 100 suitable for mounting inside a cabin of a train (not shown), in accordance with an embodiment of the present invention. The term 'train', as used herein, may refer to any vehicle that runs over railway tracks for transportation of cargo or passengers or for performing maintenance activities along a railway track. Non-limiting examples of trains may include passenger trains, intercity trains, high-speed rail services, higher-speed rail services, rapid transit trains, monorails, people movers, freight trains and so on. The train may consist of only a locomotive, or a locomotive attached to one or more railcars. The locomotive houses a driver-machine interface and one or more onboard equipment that help a driver of the train to manage operations of the train. In the present invention, the driver-assist system 100 includes a Heads-Up Display (HUD) 105 communicatively coupled to the one or more onboard equipment 110. The HUD 105 is a head-mounted wearable device that enables the driver to view safety-critical information in real-time irrespective of direction of gaze. In an embodiment, the onboard equipment is a Driver-Machine Interface. In another embodiment the onboard equipment is an onboard vital computer. It must be understood by a person skilled in the art that the onboard equipment may leverage hardware and/or software capabilities of different equipment onboard the train to implement the functionalities described herein. The HUD 105 may be configured to communicate with the onboard equipment via wired or wireless communication protocols including, but not limited to, Bluetooth, Wi-Fi, Li-Fi and other proprietary protocols that adhere to railway safety standards.

Each of the onboard equipment comprises a plurality of interfaces for communicating with the one or more subsystems and the HUD 105. In present embodiment, the onboard equipment 110 is an onboard vital computer that comprises a processing unit 175, a memory 180, a GUI 182 and a communication unit 184. The GUI 182 may include a web-based interface, a web-based downloadable application interface, and so on. The communication unit 184 may consist of a transmitter (not shown), a receiver (not shown) and a communication port (not shown) for connecting to the network interface directly or through access points. More specifically, the communication unit 184 enables the onboard equipment 110 to communicate with the head mounted unit and one or more railway subsystems such as Automatic Train Operation subsystem, Automatic Train Protection subsystem etc.

The processing unit 175, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 175 may also include embedded controllers, such as generic or programmable logic devices or arrays, application specific integrated circuits, single-chip computers, and the like. In general, the processing unit 175 may comprise hardware elements and software elements. The processing unit 175 may be configured for multithreading for hosting different calculation processes at the same time and executing the either in parallel or switching between active and passive calculation processes.

The memory 180 may be volatile memory and non-volatile memory. The memory 180 may be coupled for communication with the processing unit 175. The processing unit 175 may execute a data producer module 195 in the form of machine-readable instructions and/or code stored in the memory 180, for processing operational data obtained from at least one of the subsystems to determine safety-critical data, generating one or more commands based on the safety-critical data determined, and transmitting the one or more commands for execution, to the HUD 105. The execution of the one or more commands causes the HUD 105 to display safety-critical data in real-time.

A variety of computer-readable storage media may be stored in and accessed from the memory 180. The memory 180 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like.

The onboard equipment 110 further comprises a plurality of communication (Input/Output) interfaces 185-1, 185-2...185-9 for receiving operational data associated with the train from subsystems 190-1, 190-2...190-9 (henceforth collectively referred as subsystems 190) in real-time and for providing operational instructions, generated by the apparatus 105, to the subsystems 190 for execution at the subsystems 190. For the sake of brevity and for ease of explanation, the present embodiment is explained by considering a Driver Machine Interface (DMI) 190-1, a Juridical Recording Unit (JRU) 190-2, a radar system 190-3, a Odometer Pulse Generator (OPG) 190-4, a balise 190-5, a Passenger Information System/Transit Management System (PIS/TMS) 190-6, a diagnostics system 190-7, driver's cab controls 190-8 and a train control 190-9 as the subsystems 190. Accordingly, the plurality of communication interfaces 185-1, 185-2...185-9 include a Driver Machine Interface (DMI) interface 185-1, a Juridical Recording Unit (JRU) interface 185-2, radar interface 185-3, Odometer Pulse Generator (OPG) interface 185-4, a balise interface 185-5, a Passenger Information System/Transit Management System (PIS/TMS) interface 185-6, a diagnostics interface 185-7, a driver's cab interface 185-8 and a train control interface 185-9. Each of the communication interfaces 185-1, 185-2...185-9 may comprise a transmitter (not shown), a receiver (not shown) and a communication port (not shown) that enables the onboard equipment 110 to communicate with the respective subsystem 190.

The DMI interface 185-1 is configured to enable the onboard equipment 110 to communicate with the DMI 190-1 on the train. The onboard equipment 110 may receive inputs or control commands provided by a driver the DMI 190-1, over the DMI interface 185-1. The onboard equipment 110 may also provide feedback to the driver through the DMI 190-1, over the DMI interface 185-1. In one example, the DMI 190-1 may be an LCD touch screen for receiving touch gestures. For example, the human operator may provide touch gestures for changing a mode of operation of the train from manual to semi-automatic. The LCD touch screen may also provide visual indications relating to a status of operation of the train. For example, the status may include a speed of the train, a permitted speed of the train, an upcoming station, an estimated time of arrival at the upcoming station and so on. The visual indications may also relate to alarms or instructions to the driver of the train.

The Juridical Recording Unit (JRU) interface 185-2 is configured to enable the onboard equipment 110 to communicate with the JRU 190-2. The JRU 190-2 is a device which facilitates collecting, storing and retrieving vital information associated with the train. The vital information may include audio and video signals related to events associated with the train. For example, the video signals are collected from CCTV units installed across the train. The onboard equipment 110 may store and retrieve information stored in the JRU 190-2, through the JRU interface 185-2.

The radar interface 185-3 is configured to enable the onboard equipment 110 to receive a speed of the train from the radar system 190-3. In one example, the radar system 190-3 may be mounted on the train and is configured to measure a speed of the train, for example, based on Doppler effect.

The Odometer Pulse Generator (OPG) interface 185-4 is configured to enable the onboard equipment 110 to communicate with the OPG 190-4. The OPG 190-4 may be located on an axle of the train and may measure a distance covered by the train.

The balise interface 185-5 is configured to enable the onboard equipment 110 to communicate with at least one of a balise transmission module provided on the train or a balise 190-5 provided on the railway track. Each balise may be associated with unique track coordinates, that enable the onboard equipment 110 to determine an accurate location of the train.

The Passenger Information System/ Transit Management System (PIS/TMS) interface 185-6 is configured to enable the onboard equipment 110 to communicate with the PIS/TMS 190-6. In one example, the PIS/TMS interface 185-6 receives operational data such as distance to an upcoming station, estimated time of arrival at the upcoming station, duration of stoppage at the upcoming station from the PIS/TMS 190-6.

The diagnostics interface 185-7 is configured to enable the onboard equipment 110 to communicate with the diagnostics system 190-7 on the train. The diagnostics system 190-7 may record and store diagnostic information such as faults associated with the train, operational data associated with the train, correlations between the faults, an operating state of the train and so on.

The driver's cab interface 185-8 is configured to enable the onboard equipment 110 to communicate with the driver's cab control 190-8 provided in the driver's cab. For example, the onboard equipment 110 may receive operational data associated with the driver's cab control 190-8 associated with opening and closing of doors, operation of lights, communication systems and so on. In another example, the operational data may include a mode of operation of the train. The mode of operation may be one of manual, automatic, fully automatic, semi-automatic and extended automatic.

The train control interface 185-9 is configured to enable the onboard equipment 110 to communicate with one or more train controls 190-9. The train controls 190-9 is configured for providing control signals for driving the train and also for actuating brakes. The brakes may include an electric braking system for primary braking and a pneumatic braking system for standby or secondary purposes.

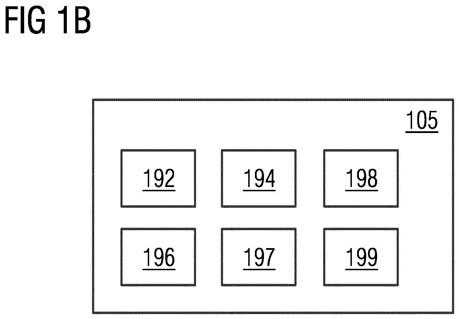

FIG 1B illustrates functional blocks of the HUD 105, in accordance with an embodiment of the present invention. The HUD 105 includes a data interface 192, a translucent display 194, a controller unit 196 and an optics unit 197. The data interface 192 enables the HUD 105 to receive commands transmitted by the onboard equipment 110. The controller unit 196 is configured for generating visuals indicative of the safety-critical data, based on the one or more commands received. The optics unit 197 is configured to display the visuals generated by the controller unit 196, on the translucent display 194. The optics unit 197 may include one or more lenses configured to project an image corresponding to the visuals in the field of vision of the user. In an embodiment, the controller unit 196 is a microcontroller that generates vector data representative of the visuals, based on the one or more commands received from the onboard equipment 110. As the functions of a HUD 105 is apparent to a person skilled in the art, details of functioning of the HUD 105 are not further elaborated herein.

In a further embodiment, the HUD 105 further includes one or more physiological sensors 198 and a feedback unit 199. The feedback unit 199 is configured to identify one or more hazards based on one or more physiological signs associated with the driver, detected by the one or more physiological sensors 198. Further, the feedback unit 199 configures a feedback signal based on the identified one or more hazards. The feedback signal is further concurrently outputted with the visuals on the translucent display 194. In an embodiment, the feedback signal is a tactile signal, such as vibration. In another embodiment, the feedback signal is an audio signal.

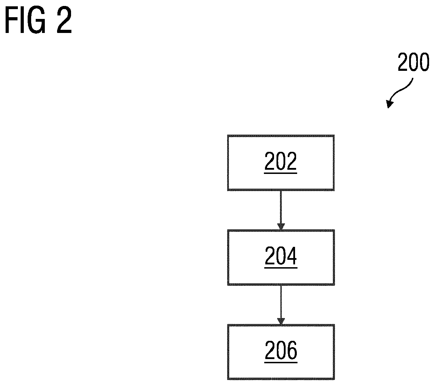

FIG 2 shows a flowchart of a method 200 for enabling the driver-assist system 100 for monitoring operations of a train in real-time, in accordance with an embodiment of the present invention. The method is implemented on the onboard equipment 110. As mentioned earlier, the onboard equipment 110 is communicatively coupled to the HUD 105 and the one or more subsystems 190 associated with the train.

At step 202, operational data obtained at least one of the subsystems 190 is processed to determine safety-critical data. In an embodiment, processing the operational data may include preprocessing, filter and/or analyzing the operational data to identify safety-critical data. The safety-critical data may be in different forms such as real-time quantitative values, categorical variables, trends, text embeddings, safety warnings etc. For example, specific operational data, say speed of the train obtained from radar interface 185-3, may be compared against predefined upper or lower limits associated with the parameter indicated by the operational data, to determine if the parameter is within safe limits. Further, a value of the categorical variable, `Safe' or 'Unsafe', may be identified for the parameter. In a further example, if the value is 'Unsafe', a further safety warning corresponding to the value may be identified from a look up table. For example, if the parameter is speed of the train, and if the speed exceeds 200 kmph defined for a specific route, the safety warning may be "Warning: Exceeded speed limit for the zone". In another example, the operational data is processed to identify a trend, e.g., a time-series graph, associated with the parameter.

At step 204, one or more commands are generated based on the safety-critical data determined. The one or more commands is associated with configuring the HUD 105 to display the safety-critical data in a predefined format. The one or more commands are generated in predefined formats based on factors such as type or criticality of the safety-critical data (e.g., alerting the driver to perform an action, informing the driver of an event indicated by the safety-critical data, real-time value of a parameter etc.), preferences (e.g., language, font size, font color) predefined by the driver, etc. The format may be predefined for each type of safety-critical data and stored in the memory 180. The one or more commands specify configuration parameters related to size, color, transparency, contrast, refresh rate, layout, alignment, etc., associated with visuals to be generated for displaying the safety-critical data. It must be understood that the one or more commands are also embedded with values indicative of the safety-critical data, along with the configuration parameters.

At step 206, the one or more commands are transmitted to the HUD 105 for execution. For example, if the HUD 105 is communicatively coupled to the onboard equipment 110 via Bluetooth, the one or more commands are packaged into messages that comply with Bluetooth protocol. The execution of the one or more commands causes the HUD 105 to display the safety-critical data in real-time.

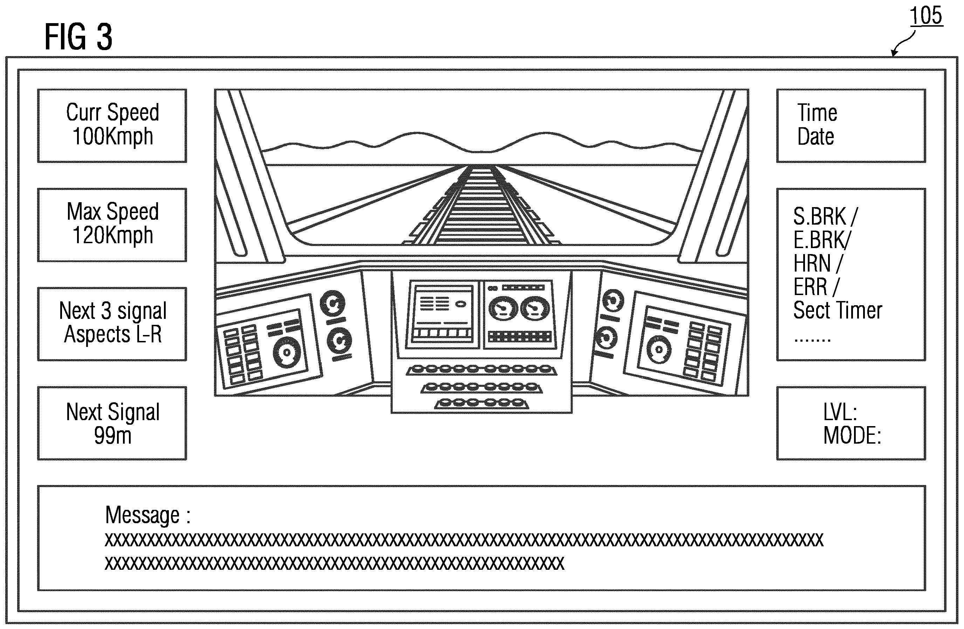

The HUD 105 further receives the one or more commands transmitted by the onboard equipment 110. Further, the HUD 105 generates visuals indicative of the safety-critical data. In particular, the HUD 105 preprocesses the messages to identify the configuration parameters and the safety-critical data. Further, the controller unit 196 of the HUD 105 generates vector data corresponding to the visuals based on the identified configuration parameters and the safety-critical data. The vector data is further provided to the optics unit 197 of the HUD 105 for projecting or displaying onto the translucent display 194. FIG 3 shows an exemplary illustration of visuals displayed on the HUD 105. As seen, the visuals include safety-critical data in the form of real-time values (e.g., Current speed 10 kmph), warnings (e.g., Max speed 120 kmph), alerts, events etc.

As mentioned earlier, the HUD 105 also includes the one or more physiological sensors 198 to detect physiological signs associated with the driver. In another implementation, the physiological sensors 198 may be a physically separate sensing unit that is communicatively coupled to the HUD 105. For example, an image-based sensing unit installed inside the cabin may be configured to capture the images of the driver regularly. Further, the sensing unit may process the images using image processing techniques to assess the physiological signs. The physiological signs are indicative of conditions such as fatigue, drowsiness etc., that are indicative of hazards. Non-limiting examples of physiological signs include eye blink rate, gaze direction, blood pressure, skin color, etc. In an example, if the eye blink rate is below a certain threshold limit, it may indicate that the driver is drowsy.

Further, the feedback unit 199 of the HUD 105 identifies one or more hazards based on one or more physiological signs associated with the driver and configures a feedback signal based on the one or more identified hazards. The feedback signal alerts the driver to pay attention to the safety-critical data displayed on the translucent display 194. In an embodiment, the feedback signal comprises at least one of a tactile signal and an audio signal. For example, the tactile signal may cause the HUD 105 to vibrate. The audio signal may include an audio indicative of the safety-critical data. It must be understood by a person skilled in the art that the audio may vary from predefined tones to an audio format of the safety-critical data generated using text to audio conversion algorithms.

The feedback signal is concurrently outputted with the visuals on translucent display 194. The term 'concurrently outputted' as used herein refers to outputting the feedback signal when a specific visual is displayed on the translucent display 194. For example, if the visuals are indicative of an 'alert' which requires the user to perform an action, the audio may include a predefined audio that indicates the criticality of the alert. The driver may choose to dismiss the feedback signal manually, for example, by way of gestures, audio or speech inputs. Alternatively, the feedback signal may be automatically dismissed when the safety-critical data is no longer valid, for example, if the speed has resumed to normal range from a dangerously high value. Further, the volume of the audio may also increase if the alert remains unaddressed after a predefined period.

Advantageously, the present invention enables the driver of the train to focus on the track ahead without the need to continuously shift gaze between the driver machine interface and track. The present invention also allows the driver to focus only on critical information thereby relieving them from effects of information fatigue resulting from focusing on the driver machine interface. Additionally, the present invention also causes the driver to be alert by way of the feedback signal provided along with the visuals.

While the present invention has been described in detail with reference to certain embodiments, it should be appreciated that the present invention is not limited to those embodiments. In view of the present disclosure, many modifications and variations would be present themselves, to those skilled in the art without departing from the scope of the various embodiments of the present invention, as described herein. For example, a windshield of a train may be modified similar to the HUD described above, to facilitate the driver to view critical information directly on the windshield, while simultaneously observing the track ahead.

The scope of the present invention is, therefore, indicated by the following claims rather than by the foregoing description. All changes, modifications, and variations coming within the meaning and range of equivalency of the claims are to be considered within their scope.

### List of reference numerals

100 driver-assist system
105 Heads-up display
110 onboard equipment
175 processing unit
180 memory
182 GUI
184 communication unit
185-1, 185-2...185-9 communication interfaces
190-1, 190-2...190-9 subsystems
192 data interface
194 translucent display
195 data producer module
196 controller unit
197 optics unit
198 physiological sensors
199 feedback unit

## Claims

1. An onboard equipment (110) configured for mounting inside a cabin of a train, the onboard equipment (110) comprising:
one or more communication interfaces (185) for enabling connection with a heads-up display (105) and one or more subsystems (190) onboard the train;
a memory (180); and
a processing unit (175) configured to execute one or more instructions stored in the memory (180), to:
process operational data obtained from at least one of the subsystems (190) to determine safety-critical data;
generate one or more commands based on the safety-critical data determined;
transmit, to the heads-up display (105), the one or more commands for execution, wherein the execution of the one or more commands causes the heads-up display (105) to display the safety-critical data in real-time.

2. The onboard equipment (110) according to claim 1, wherein the onboard equipment (110) is a Driver-Machine Interface.

3. The onboard equipment (110) according to claim 1, wherein the onboard equipment (110) is an onboard vital computer.

4. A driver-assist system (100) comprising:
a heads-up display (105), suitable for wearing by a driver of a train; and
an onboard equipment (110) comprising:
one or more communication interfaces (185) for enabling connection with a heads-up display (105) and one or more subsystems (190) onboard the train;
a memory (180); and
a processing unit (175) configured to execute one or more instructions stored in the memory (180), to:
process operational data obtained from at least one of the subsystems (190) to determine safety-critical data;
generate one or more commands based on the safety-critical data determined;
transmit, to the heads-up display (105), the one or more commands for execution, wherein the execution of the one or more commands causes the heads-up display (105) to display the safety-critical data in real-time.

5. The system (100) according to claim 4, wherein the onboard equipment (110) is a Driver-Machine Interface.

6. The system (100) according to claim 4, wherein the onboard equipment (110) is an onboard vital computer.

7. The system (100) according to claim 4, wherein the heads-up display (105) comprises:
a data interface (192) for receiving the one or more commands transmitted by the onboard equipment (110) ;
a translucent display (194);
a controller unit (196) configured to generate visuals indicative of the safety-critical data, based on the one or more commands received;
an optics unit (197) configured to display the visuals generated by the controller unit (196), on the translucent display (194).

8. The system (100) according to claim 7, wherein the heads-up display (105) further comprises:
one or more physiological sensors (198); and
a feedback unit (199) configured to:
identify one or more hazards based on one or more physiological signs associated with the driver, detected by the one or more physiological sensors (198);
configure a feedback signal based on the identified one or more hazards; and
concurrently output the feedback signal with the visuals on the translucent display (194).

9. The system (100) according to claim 8, wherein the feedback signal comprises at least one of a tactile signal and an audio signal.

10. A method comprising:
processing, by an onboard equipment (110) mounted inside a cabin of a train, operational data obtained from at least one subsystem onboard the train to determine safety-critical data;
generating, by the onboard equipment (110), one or more commands based on the safety-critical data determined; and
transmitting, by the onboard equipment (110), the one or more commands for execution, to a heads-up display (105), wherein the execution of the one or more commands causes the heads-up display (105) to display the safety-critical data in real-time.

11. The method according to claim 10, further comprising:
generating, by the heads-up display (105), visuals indicative of safety-critical data, by the heads-up display (105) based on the one or more commands received from the onboard equipment (110).

12. The method according to claim 10, further comprising:
identifying, by the heads-up display (105), one or more hazards based on one or more physiological signs associated with the driver;
configuring, by the heads-up display (105), a feedback signal based on the identified one or more hazards; and
concurrently outputting, by the heads-up display (105), the feedback signal with the visuals.

13. The method according to claim 12, wherein the feedback signal comprises at least one of a tactile signal and an audio signal.
